(19) Europäisches Patentamt
European Patent Office
Office européen des brevets

(11) Publication number: **0 142 979**
**B1**

(12) # EUROPEAN PATENT SPECIFICATION

(45) Date of publication of patent specification: **01.02.89**

(51) Int. Cl.$^4$: **C 07 C 69/736,** C 07 C 67/31, C 07 C 59/72, C 07 C 51/09

(21) Application number: **84307864.3**

(22) Date of filing: **14.11.84**

(54) **Improvements in the production of halocyclopropyl-substituted phenoxyalkanoic acids and esters.**

(30) Priority: **15.11.83 GB 8330407**

(43) Date of publication of application:
**29.05.85 Bulletin 85/22**

(45) Publication of the grant of the patent:
**01.02.89 Bulletin 89/05**

(84) Designated Contracting States:
**AT BE CH DE FR IT LI LU NL SE**

(56) References cited:
**GB-A-1 385 828**

**SYNTHESIS, no. 11, november 1977, pages 783-784, Georg Thieme Verlag, Stuttgart, DE; M. FEDORYNSKI: "Rections of organic anions; LXXVI. A convenient procedure for the generation of bromochlorcarbene and preparation of gem-bromochlorocyclopropane derivatives"**

**The file contains technical information submitted after the application was filed and not included in this specification**

(73) Proprietor: **STERWIN AG.**
**Zeughausgasse 9**
**CH-6300 Zug (CH)**

(72) Inventor: **Thorpe, John Graham**
**22 Melton Drive Melton Park**
**Gosforth Newcastle-upon-Tyne NE3 5QB (GB)**
Inventor: **Peel, Richard**
**43, Archibald Street**
**Gosforth Newcastle-upon-Tyne NE3 1EB (GB)**

(74) Representative: **Keltie, David Arthur et al**
**Baron & Warren 18 South End Kensington**
**London W8 5BU (GB)**

**EP 0 142 979 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the production of halocyclopropyl-substituted phenoxyalkanoic acids and esters thereof.

In British Patent Specification No. 1 385 828 there are described a number of halocyclopropyl-substituted phenoxyalkanoic acids as well as their esters. Amongst these are a number of such acids in which the halocyclopropyl ring is directly connected to the phenoxy ring and the alkanoic moiety of the structure contains a minimum of four carbon atoms. Acids of this type have been found to be of pharmacological interest as they possess hypocholesteremic and hypotriglyceridemic activity and are therefore of interest in the treatment of atherosclerotic conditions associated with elevated serum cholesterol and triglyceride levels.

One method of preparation described in British Patent No. 1 385 828 involves the preliminary preparation of the p-(2,2-dihalocyclopropyl) phenols: these materials are of limited availability and are relatively expensive. It is accordingly an object of the present invention to provide an improved method of preparation of such acids and their esters from readily available starting materials.

Instead of first producing a p-(2,2-dihalocyclopropyl)-phenol another method for obtaining certain of the compounds was also proposed by condensing a 2-phenoxy propionate and a haloacetyl chloride in the presence of aluminium chloride or other Lewis acid catalyst to produce a ketophenoxy ester followed by reduction to the corresponding carbinol. The carbinol was then dehydrated using a dehydrating agent in an inert solvent, the latter acting as an entraining agent whilst heating to produce a styrene which has then been reacted with carbene or a substituted carbene generated *in situ*. The introduction of the cyclopropyl group as the last stage of the process is considered preferable but the process has nevertheless suffered from the drawback of giving a low overall yield as well as rather difficult reaction conditions. Alternative methods have therefore been sought and have resulted in the ultimate discovery of a procedure which gives a greater than ten-fold increase in the yield obtained.

The acids and esters to which the present invention relates have the general formula

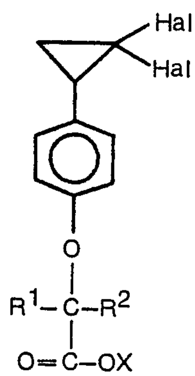

(I)

in which each Hal represents a halogen atom, each of $R^1$ and $R^2$ represents an alkyl group having at least one but not more than three carbon atoms and X represents hydrogen or C1—C5 alkyl. In the present procedure the acids (I) are produced by hydrolysis of the corresponding esters.

According to the present invention there is provided a process for the production of an alkyl ester having the general formula

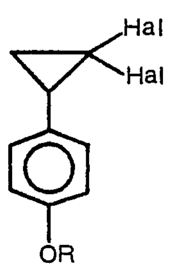

(II)

in which each Hal is a halogen atom, preferably chlorine, and R is a group having the general formula:

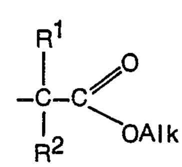

(III)

in which Alk represents an alkyl group having one to five carbon atoms and each of $R^1$ and $R^2$ is an alkyl group having one to three carbon atoms, preferably a methyl group, wherein an alkyl ester having the general formula

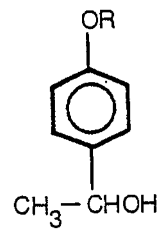

(IV)

in which R is as above defined is converted to a substituted styrene having the general formula

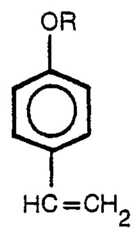

(V)

in which R is as above defined, the concomitantly formed hydrohalide of the organic base is removed and the substituted styrene is then converted to the compound of formula (II) by a carbene addition, characterised in that the alkylester (IV) is treated, in solution in a haloform, with a phosphorous halide to replace the —OH group with a halogen atom and then with an organic base to produce the substituted styrene (V), and in that a strong alkali and a phase transfer catalyst are added to the reaction mixture to initiate the carbene addition, the carbon being derived from the haloform solvent.

It will be understood that there is thus obtained a product having the —O-Alk group. When it is desired to obtain the corresponding free acid such ester is saponified with an alkali metal hydroxide and the resulting solution, normally an aqueous solution, is acidified with a strong acid.

To obtain the alkyl esters of the general formula (IV), esters having the general formula

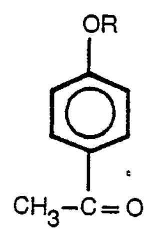

(VI)

are reduced by the gradual addition thereto of an alkali metal borohydride, preferably sodium borohydride, in a lower alkanol, preferably ethanol. Other reducing agents such as hydrogen in the prsence of palladium on charcoal may also be used.

It will be apparent that the group R having the general formula (III) may be a 2-methylpropanoic, a 2-ethylpropanoic or a 2-ethylbutanoic group.

Compounds having the general formula (VI) may be synthesised from the readily available para-hydroxyacetophenone by reaction with dialkyl ketones having not more than 7 carbon atoms and corresponding to the formula $R^1COR^2$, in which $R^1$ and $R^2$ are as above defined, in the presence of a haloform and a solution of one or more alkali metal hydroxides. This gives a carboxylic acid which may then be esterified with an alkanol such as ethanol.

In this way there may, for example, be prepared 2-(4'-acetylphenoxy)-2-methyl- and 2-ethyl-propanoic acids and these may then be esterified with methanol or ethanol in conventional manner.

The esters having the general formula (VI) are reduced to the corresponding secondary alcohols (IV) by reduction of the acetyl group. This may be effected by the action of an alkali metal borohydride on a solution of the ester dissolved in a lower alkanol and isolation of the alcohol from the resulting solution by taking advantage of its solubility in the organic phase of a toluene-brine mixture, or by another reducing agent such as hydrogen in the presence of palladium on charcoal. In this way the compounds having the general formula (IV) are obtainable.

In a preferred process accoridng to the invention compounds having the general formula (IV) are then

treated in haloform solution with a phosphorus halide, preferably phosphorus tribromide, at room temperature or below. When the reaction which occurs is substantially complete the product is treated with an organic base such as trialkylamine in order to remove the elements of hydrogen halide from the product. The resulting ethenyl compound (V) does not have to be isolated from the solution obtained, and is directly used in the next stage of the process.

In the next stage of the process the compounds having the general formula (V) are subject to a carbene addition reaction with the haloform solvent which is conveniently chloroform, in the presence of a strong alkali preferably an aqueous alkali metal hydroxide. This reaction is effected in the presence of a phase transfer catalyst. The haloform used will be present in substantial excess as the solvent used in the earlier reaction steps. The solvent is conveniently removed under reduced pressure after completion of the reaction and the resulting ester containing a 2,2-dihalocyclopropyl group can then be distilled under low pressure. With or without such distillation the ester may then be saponified using a solution of an alkali metal hydroxide in a lower alkanol and the resulting solution acidified, the acid separated and purified.

The appended example illustrates the preparation of 2-[4-(2',2'-dichlorocyclopropyl)phenoxy]-2-methyl propanoic acid by the process of the invention.

*Ethyl 2-(4-Ethenylphenoxy)-2-methylpropanoate*

Ethyl 2-[4-(1-Hydroxyethyl)phenoxy]-2-methylpropanoate (100 g) was dissolved in dry chloroform (200 mls) — dimethylformamide (2.5 mls) was then added. The solution was cooled to 0°C and a solution of phosphorus tribromide (15 mls) in dry chloroform (40 mls) was added. After stirring for 1 hour at 0—5°C followed by 1 hour at 30°C — triethylamine (70 g) was added maintaining the temperature below 30°C. After refluxing for a total of 18 hours — the chloroform solution was washed with water and twice with dilute HCl to remove the triethylamine hydrochloride formed. The chloroform solution of the ester can be processed directly in the next stage. However, if desired, the chloroform can be evaporated to give the crude styrene as an oil which can be purified by high vacuum distillation (bp 120°C/0.5 mm).

*Ethyl 2-[4-(2',2'-Dichlorocyclopropyl)phenoxy]-2-methyl Propanoate*

Ethyl 2-(4-ethenylphenoxy)-2-methylpropanoate (75 g) obtained as described above in chloroform (160 mls) containing benzyl triethyl ammonium chloride phase trans-catalyst was reacted with a solution of 50% sodium hydroxide at 40°C for five hours. The solution was cooled, diluted with water and separated. The chloroform solution was evaporated under reduced pressure to give the product as a dark oil.

The crude ester can be purified by high vacuum distillation and has bp 160—170°C/0.5 mm.

*2-[4-(2',2'-Dichlorocyclopropyl)phenoxyl]-2-methyl propanoic acid*

150 g of distilled ethyl-2-(4-(2',2'-Dichlorocyclopropyl)phenoxy]-2-methyl propanoate prepared as described above was added to methanol (550 mls) containing sodium hydroxide (25.1 g); the resulting solution was heated for 2 hours at 50—55°C. The methanol was evaporated under reduced pressure and the residue dissolved in water and extracted with hexane. The aqueous layer was acidified to pH 1 with concentrated HCl. The product was isolated by filtration and washed thoroughly with water.

Yield = 123.1 g (90%) — mp 113—117°C.

The crude acid, from the above procedure, was further purified by recrystallisation from aqueous alcohol to give the pure acid mp 116—118°C.

The starting material used in the above procedure may be prepared in the following manner:

*Ethyl 2-[4-(1-Hydroxyethyl)phenoxyl]-2-methylpropanoate*

Ethyl 2-(4-Acetylphenoxy)-2-methylpropanoate (100 g) was dissolved in ethanol (450 ml) and cooled to 0°C; sodium borohydride (7.5 g) was added over 1 hour at 0—5°C. The solution was stirred for 1 hour at 0—5°C and then for 1 hour at room temperature. Glacial acetic acid (14.3 mls) was then added over 1 hour and the solution stirred a further hour. The bulk of the ethanol was then removed under reduced pressure and the residue partitioned between toluene and water. The toluene layer was washed with water (×2) and saturated brine and the solvent removed under reduced pressure to leave the product as an oil. Yield = 90.7 g (90%).

The temperatures used in the above process all lie within the range of 0° to 60°C thus demonstrating that neither low or high temperatures have to be used. This is an important feature since it minimises the energy consumed, avoids the use of complicated and expensive equipment and thus makes the process an economic one.

Another important feature of the new process in its preferred form is the passage of the styrene produced by dehydrohalogenation direct to the step in which dihalocarbene is added on to the ethylenic linkage without intermediate isolation of the styrene. Yet another important feature is the use of a phase transfer catalyst in the dihalocarbene addtion step. The styrene is obtainable in high yields by the present procedure and has little opportunity to polymerise under its conditions of formation and use.

The yield in the above procedure, calculated on the basis of para-hydroxyacetophenone as the effective starting material, is 37% overall whereas that in the known process starting from dichlorocarbostyrene is 3.5%. This represents more than 10-fold improvement. In addition separation of isomers which has to be carried out in the known process is avoided and problems arising from the disposal of effluents

containing heavy metals is also entirely avoided.

**Claims**

1. A process for the production of an alkyl ester of a 2-(para-substituted phenoxy)-2-alkylpropionic acid having the general formula

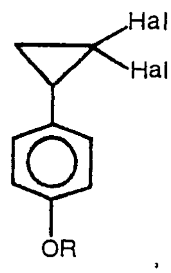

(II)

in which each Hal is a halogen atom and R is a group having the general formula

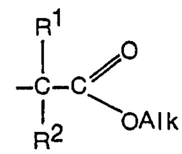

(III)

in which Alk is an alkyl group having one to five carbon atoms and each of $R^1$ and $R^2$ is an alkyl group having one to three carbon atoms wherein an alkyl ester having the general formula

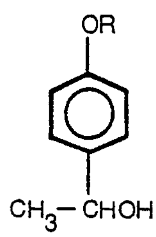

(IV)

in which R is as above defined is converted to a substituted styrene having the general formula

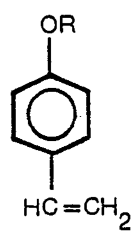

(V)

in which R is as above defined, the concomitantly formed hydrohalide of the organic base is removed and the substituted styrene is then converted to the compound of formula (II) by a carbene addition, characterised in that the alkylester (IV) is treated, in solution in a haloform, with a phosphorous halide to replace the —OH group with a halogen atom and then with an organic base to produce the substituted styrene (V), and in that a strong alkali and a phase transfer catalyst are added to the reaction mixture to initiate the carbene addition, the carbon being derived from the haloform solvent.

2. A process as claimed in claim 1, further characterised in that the phosphorous halide used is phosphorous tribromide.

3. A process as claimed in claim 1 or 2 further characterised in that each Hal represents chlorine.

4. A process as claimed in any preceding claim further characterised in that $R^1$ and $R^2$ each represent a methyl group.

5. A process as claimed in any preceding claim further characterised in that the phase transfer catalyst is benzyl triethylammonium chloride.

6. A process as claimed in any preceding claim wherein the organic base is trialkylamine.

7. A process as claimed in any preceding claim wherein the haloform is chloroform.

8. A process for the production of a 2-(para-substituted-phenoxyl)-2-alkylpropionic acid characterised

in that it comprises producing an alkyl ester of such acid having the general formula II defined in claim 1, by the process claimed in any preceding claim, saponifying such ester with an alkali metal hydroxide and acidifying the saponification product with a strong acid.

**Patentansprüche**

1. Verfahren zur Herstellung eines Alkylesters einer 2-(para-substituierten Phenoxy)-2-Alkylpropionensäure der allgemeinen Formel:

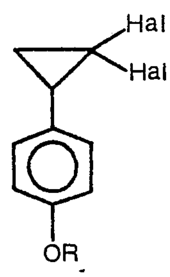
(II)

in welcher Hal eine Halogenatom und R eine Gruppe der allgemeinen Formel:

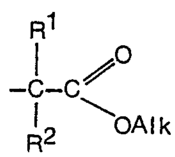
(III)

ist, in welcher Alk eine Alkylgruppe mit 1 bis 5 Kohlenstoffatomen und $R^1$ und $R^2$ jeweils eine Alkylgruppe mit 1 bis 3 Kohlenstoffatomen ist, wobei ein Alkylester der allgemeinen Formel:

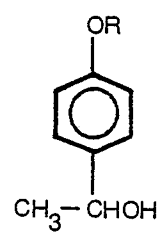
(IV)

in welcher R wie oben definiert ist, umgewandelt wird zu einem substituierten Styrol der allgemeinen Formel:

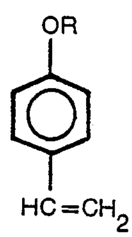
(V)

in welcher R wie oben definiert ist, das gleichzeitig gebildete Hydrohalogenid der organischen Base entfernt wird und das substituierte Styrol dann umgewandelt wird zu der Verbindung gemäß Formel (II) durch eine Carbenaddition, dadurch gekennzeichnet, daß der Alkylester (IV) in einem Haloformlösemittel mit einem Phosphorhalogenid behandelt wird, um die OH-Gruppe durch eine Halogenatom zu ersetzen und dann mit einer organischen Säure das substituierte Styrol (V) herzustellen, und daß eine konzentrierte Alkakilauge und ein Phasentransferkatalysator zu dem Reaktionsgemisch hinzugefügt wird, um die Carbenaddition zu starten, wobei der Kohlenstoff abgeleitet ist aus dem Haloformlösemittel.

2. Verfahren nach Anspruch 1, dadurch gekennzeichnet, daß das eingesetzte Phosphorhalogenid ein Phosphortribromid ist.

3. Verfahren nach Anspruch 1 oder 2, dadurch gekennzeichnet, daß jedes Hal Chlor darstellt.

4. Verfahren nach einem der vorhergenden Ansprüche, dadurch gekennzeichnet, daß $R^1$ und $R^2$ jeweils eine Methylgruppe darstellen.

5. Verfahren nach einem der vorhergenden Ansprüche, dadurch gekennzeichnet, daß der Phasentransferkatalysator ein Benzyltriethylammoniumchlorid ist.

6. Verfahren nach einem der vorhergenden Ansprüche, dadurch gekennzeichnet, daß die organische Base Trialkylamin ist.

7. Verfahren nach einem der vorhergenden Ansprüche, dadurch gekennzeichnet, daß das Haloform Chloroform ist.

8. Verfahren zur Herstellung von 2-(para substituierten Phenoxyl)-2-alkylpropionsäure dadurch gekennzeichnet, daß es die Herstellung eines Alkylesters einer Säure der allgemeinen Formel (II), die in Anspruch 1 definiert ist, durch ein Verfahren gemäß einem der vorhergehenen Ansprüche, die Verseifung eines solchen Esters mit einem Alkalimetallhydroxid und die Ansäuerung des Verseifungsprodukts mit einer konzentrierten Säure umfaßt.

**Revendications**

1. Procédé de production d'un ester alcoylique d'un acide (phénoxy à substitution para)-2 alcoyl-2 propionique ayant la formule générale

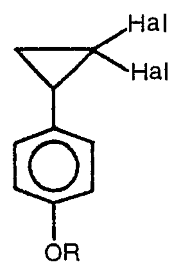

(II)

dans laquelle chaque Hal est un atome d'halogène et R est un radical ayant la formule générale

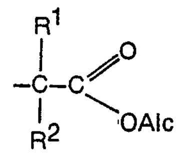

(III)

dans laquelle Alc est un radical alcoyle de un à cinq atomes de carbone, et chacun des $R^1$ et $R^2$ est un radical alcoyle ayant de un à trois atomes de carbone, selon lequel on convertit un ester alcoylique ayant la formule générale

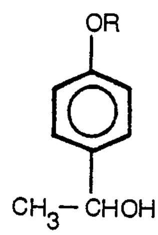

(IV)

dans laquelle R est tel que défini ci-dessus, en un styrène substitué ayant la formule générale

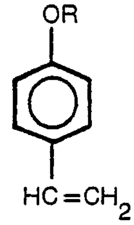

(V)

dans laquelle R est tel que défini ci-dessus, on extrait l'hydrohalogénure de la base organique qui se forme simultanément et on convertit alors le styrène substitué à l'aide d'une addition de carbène de façon à donner le composé de formule (II), caractérisé en ce qu'on traite l'ester alcoylique (IV), en solution dans un haloforme, avec un halogénure phosphoreux de façon à remplacer le radical —OH par un atome

d'halogène, puis avec une base organique de façon à produire le styrène substitué (V), et en ce qu'on ajoute au mélange de réaction une base forte et un catalyseur de transfert de phase de façon à déclencher l'addition de carbène, le carbone provenant du solvant que forme l'haloforme.

2. Procédé suivant la revendication 1, caractérisé en outre en ce que l'halogénure phosphoreux utilisé est du tribromure phosphoreux.

3. Procédé suivant la revendication 1 ou 2, caractérisé en outre en ce que chaque Hal représente le chlore.

4. Procédé suivant l'une quelconque des revendications précédentes, caractérisé en outre en ce que $R^1$ et $R^2$ représentent chacun un radical méthyle.

5. Procédé suivant l'une quelconque des revendications précédentes, caractérisé en outre en ce que le catalyseur de transfert de phase est le chlorure de benzyltriéthylammonium.

6. Procédé suivant l'une quelconque des revendications précédentes, selon lequel la base organique est la trialcoylamine.

7. Procédé suivant l'une quelconque des revendications précédentes, selon lequel l'haloforme est le chloroforme.

8. Procédé de production d'un acide (phénoxy à substitution para)-2 alcoyl-2 propionique, caractérisé en ce qu'il consiste à produire un ester alcoylique d'un tel acide ayant la formule générale (II) définie dans la revendication 1, à l'aide du procédé revendiqué dans l'une quelconque des revendications précédentes, à saponifier un tel ester à l'aide d'un hydroxyde de métal alcalin et à acidifier le produit de la saponification à l'aide d'un acide fort.